# FASCICULE DE BREVET EUROPEEN

(11) **EP 0 825 875 B1**
(45) Date de publication et mention de la délivrance du brevet: **21.06.2000**
(21) Numéro de dépôt: 96915068.9
(22) Date de dépôt: 22.04.1996
(51) Int. Cl.: A61K 39/39

(54) **COMPOSITION THERAPEUTIQUE COMPRENANT UN ANTIGENE OU UN GENERATEUR IN VIVO D'UN COMPOSE COMPRENANT UNE SEQUENCE D'ACIDES AMINES**
PHARMAZEUTISCHE ZUSAMMENSETZUNG ENTHALTEND EIN ANTIGEN ODER EINEN IN VIVO-ERZEUGER EINER VERBINDUNG MIT EINER AMINOSÄURESEQUENZ
THERAPEUTIC COMPOSITION COMPRISING AN ANTIGEN OR AN IN VIVO GENERATOR FOR GENERATING A COMPOUND COMPRISING AN AMINO ACID SEQUENCE

(30) Priorité: 20.04.1995 FR 9504739
(43) Date de publication de la demande: 04.03.1998
(73) Titulaire: SOCIETE D'EXPLOITATION DE PRODUITS POUR LES INDUSTRIES CHIMIQUES, S.E.P.P.I.C., 75321 Paris Cédex 07 (FR)
(72) Inventeur: GANNE, Vincent, 94210 La Varenne-Saint-Hilaire (FR)
(74) Mandataire: Conan, Philippe Claude
(86) Numéro de dépôt international: FR9600609
(87) Numéro de publication internationale: WO9632964

(56) Documents cités:
- EP-A- 0 224 260
- FR-A- 2 672 495
- FR-A- 2 674 755
- CHEMICAL ABSTRACTS, vol. 105, no. 23, 8 Décembre 1986 Columbus, Ohio, US; abstract no. 202906, BELOKRYLOV, G. A.: "The immunostimulating property of aspartic acid" XP002010599 & BYULL. EKSP. BIOL. MED. (1986), 102(8), 213-15,

## Description

La présente invention concerne une composition thérapeutique comprenant au moins un antigène, notamment un antigène d'origine virale, bactérienne ou parasitaire ou au moins un générateur in vivo d'un composé comprenant une séquence d'acides animés, et au moins un adjuvant. Selon un autre aspect de l'invention, celle-ci concerne l'utilisation nouvelle d'un sel pharmaceutiquement acceptable et soluble dans l'eau en tant qu'adjuvant.

L'utilisation d'adjuvants dans des compositions thérapeutiques du type vaccin est connue depuis longtemps. Ces adjuvants ont pour objet principal de permettre l'augmentation de la réponse immunitaire spécifique.

Ces adjuvants sont de nature diverse. Ils peuvent par exemple consister en des liposomes, des phases huileuses, par exemple du type adjuvants de Freund, généralement utilisées sous forme d'une émulsion avec une phase aqueuse, ou, de manière plus courante, en des sels minéraux insolubles dans l'eau. Ces sels minéraux peuvent consister par exemple en l'hydroxyde d'aluminium, le sulfate de zinc, l'hydroxyde de fer colloïdal, le phosphate de calcium ou le chlorure de calcium. L'hydroxyde d'aluminium (Al(OH)₃) est l'adjuvant le plus couramment utilisé. Ces adjuvants sont décrits notamment dans l'article de Rajesh K. Gupta et al "Adjuvants, balance between toxicity and adjuvanticity", Vaccine, vol. 11, Issue 3, 1993, pages 993-306.

Les adjuvants mentionnés ci-dessus présentent l'inconvénient d'une efficacité limitée. Par ailleurs, ils peuvent induire une certaine toxicité vis à vis des sujets traités. Plus particulièrement, lorsque ces compositions thérapeutiques sont injectées, on observe la formation de lésions et d'autres réactions locales telles que des granulomes au niveau du point d'injection. Ces inconvénients sont moins fortement marqués lorsque l'adjuvant est l'hydroxyde d'aluminium. C'est pourquoi ce dernier composé est l'un des adjuvants les plus couramment utilisés. Cependant, depuis peu, tous les composés à base d'aluminium et par conséquent l'hydroxyde d'aluminium sont suspectés d'être un facteur favorisant l'apparition de certaines maladies, telles des dysfonctionnements rénaux ou la maladie d'Alzheimer. En outre, il est connu que l'hydroxyde d'aluminium n'induit efficacement qu'une immunité humorale, et non une immunité cellulaire.

Un premier objet de l'invention consiste en des compositions thérapeutiques comprenant un adjuvant permettant une augmentation de la réponse immune au moins égale à celle conférée par l'hydroxyde d'aluminium, sans engendrer de lésions ou de réactions locales du type granulomes et qui ne soit pas suceptible de favoriser l'apparition de maladies chez le sujet traité.

Dans le cadre de la présente invention, la réponse immune dont l'augmentation est favorisée par l'adjuvant est une réponse spécifique qui s'exerce vis-à-vis de la substance immunisante.

Selon un autre aspect, l'invention a pour objet une composition thérapeutique comprenant un adjuvant induisant efficacement aussi bien une immunité cellulaire qu'une immunité humorale.

Selon encore un autre aspect, l'invention concerne l'utilisation de nouveaux adjuvants pour la préparation d'une composition thérapeutique.

Selon encore un autre aspect, l'invention concerne de nouvelles compositions adjuvantes utiles pour la préparation de compositions thérapeutiques.

La présente invention concerne ainsi une composition thérapeutique comprenant (i) au moins un antigène ou au moins un générateur in vivo d'un composé comprenant une séquence d'acides aminés et (ii) au moins un adjuvant, caractérisée en ce que ledit adjuvant consiste en au moins un sel pharmaceutiquement acceptable et soluble dans l'eau, ledit sel étant constitué d'un anion organique et d'un cation métallique choisi dans le groupe constitué par le manganèse, le calcium et le zinc.

Le manganèse est un métal tout particulièrement préféré dans le cadre de la présente invention. Il a en effet été constaté que les sels pharmaceutiquement acceptables et solubles dans l'eau selon l'invention comprenant un cation Mn²⁺ permettaient l'induction d'une réponse immune particulièrement importante, tout en présentant une toxicité faible.

Un sel soluble dans l'eau, au sens de la présente invention, peut être tel que sa solubilité dans l'eau est supérieure ou égale à 10 g/l, de préférence comprise entre 10 et 2000 g/l.

L'anion organique constitutif dudit sel pharmaceutiquement acceptable est avantageusement un anion d'un composé comprenant au moins un groupe fonctionnel oxygéné, de préférence un groupe phosphorique -PO₄H₂ ou un groupe carboxylique -COOH.

L'acide glycérophosphorique est un anion à groupe phosphorique préféré.

Les anions comprenant au moins un groupe carboxylique préférés sont dérivés de composés choisis parmi :
- les oses acides, de préférence les oses acides ayant de 5 à 7 atomes de carbone, plus préférentiellement ceux ayant 6 ou 7 atomes de carbone,
- les acides mono ou polycarboxyliques,
- les acides aminés.

Les acides mono ou polycarboxyliques préférés sont l'acide fumarique et les composés de formule (I) : 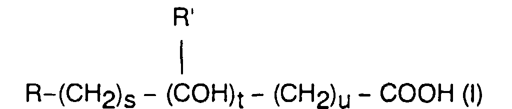 où :
R représente COOH, CH₃ CO, CH₃ ou CH₂OH,
R' représente H ou COOH
et s, t et u, identiques ou différents sont compris entre O et 3.

Des composés de formule (I) préférés sont l'acide acétique, l'acide lactique, l'acide tartrique, l'acide malique, l'acide citrique ou l'acide pyruvique.

Au sens de la présente invention, un ose acide consiste en un ose comprenant au moins une fonction carboxylique, un ose étant un glucide constitué par les sucres réducteurs. Ces oses acides sont avantageusement des dérivés d'aldoses, obtenus par oxydation de la fonction alcool primaire ou de la fonction aldéhydique en fonction carboxylique. De tels composés peuvent tout particulièrement consister en l'acide gluconique, l'acide glucuronique, l'acide fructoheptonique, l'acide gluconoheptonique, l'acide glucoheptonique. Lorsque l'anion organique est dérivé d'un acide aminé, cet acide aminé peut être un acide α-aminé tel l'acide glutamique, la méthionine et, tout particulièrement, l'acide aspartique.

Un sel pharmaceutiquement acceptable, soluble dans l'eau selon l'invention peut comprendre un cation divalent tel que défini précédemment, associé à un anion organique ou à plusieurs anions organiques de nature différente. Ainsi, à titre d'exemple, un cation divalent tel le cation du calcium peut être associé à un anion dérivé de l'acide gluconoheptonique et un anion dérivé de l'acide gluconique.

Un adjuvant soluble dans l'eau tout particulièrement préféré dans le cadre de la présente invention consiste en le gluconate de manganèse.

Une composition thérapeutique selon la présente invention peut comprendre entre 0,01 et 1000 mg/ml, de préférence entre 0,1 et 150 mg/ml d'un adjuvant tel que défini ci-dessus.

Une composition thérapeutique selon l'invention peut être préparée par simple mélange d'une suspension aqueuse contenant l'antigène ou ledit générateur in vivo avec une solution aqueuse du sel défini ci-dessus.

La composition thérapeutique selon l'invention, outre l'adjuvant consistant en un sel pharmaceutiquement acceptable soluble dans l'eau, tel que défini ci-dessus, peut également comprendre un adjuvant huileux. Dans un tel cas, la composition thérapeutique selon l'invention se présente avantageusement sous la forme d'une émulsion associant au moins une phase aqueuse et au moins une phase huile.

Cette émulsion peut être du type eau dans huile (E/H), ou, prérentiellement du type huile dans eau (H/E), eau dans huile dans eau (E/H/E) ou microémulsion. Une telle émulsion peut être préparée selon les méthodes classiques de préparation d'une émulsion, notamment selon les procédés décrits dans les demandes de brevets EP-A-489.181 et EP-A-481.982. Ainsi, on peut émulsionner sous agitation l'huile constitutive de la phase huile avec la phase aqueuse constituée d'une solution ou d'une suspension aqueuse contenant l'antigène .

Une émulsion selon l'invention peut comporter, en poids, de 0,5 % à 99,5 % de phase huile pour 99,5 % à 0,5 % de phase aqueuse, de préférence, de 5 à 95 % de phase huile pour 95 à 5 % de phase aqueuse et, plus préférentiellement, de 25 à 75 % de phase huile pour 75 à 25 % de phase aqueuse. L'émulsion doit être stable de préférence pendant au moins 12 mois quand elle est stockée à 4°C.

L'adjuvant huileux peut être une huile minérale, une huile non minérale ou un mélange d'une huile minérale et d'une huile non minérale. Lesdites huiles minérales peuvent être naturelles ou synthétiques. Lesdites huiles non minérales peuvent être d'origine végétale, animale ou synthétique. Avantageusement, les huiles non minérales sont métabolisables. Toutes ces huiles sont dépourvues d'effets toxiques à l'égard de l'organisme hôte chez lequel la composition de l'invention est administrée. Elles sont de préférence liquides à la température de stockage (environ +4°C) ou, au moins, permettent de donner des émulsions liquides à cette température. Une huile minérale avantageuse selon l'invention peut consister en une huile comprenant une chaîne carbonée linéaire ayant un nombre d'atomes de carbone de préférence supérieur à 16, et exempte de composés aromatiques. De telles huiles peuvent être par exemple celles commercialisées sous la désignation "MARCOL 52" (produit par Esso France) ou "DRAKEOL 6VR" (produite par Penreco USA).

A titre d'huiles non minérales synthétiques, on peut citer les polyisobutènes, les polyisopropènes, les esters d'alcools et d'acides gras, tels que, par exemple, l'oléate d'éthyle, le myristate d'isopropyle, les mono, di ou triglycérides, les esters de propylène glycol, les glycérides partiels, tels les glycérides d'huile de maïs comme ceux commercialisés par la société SEPPIC sous la désignation LANOL TM, la maïsine ou l'oléate d'oléyle. Parmi les huiles végétales, on peut citer des huiles insaturées riches en acide oléique qui sont biodégradables, par exemple les huiles d'arachide, d'olive, de sésame, de soja ou de germes de blé.

Les huiles animales peuvent consister notamment en le squalène, le squalane ou l'huile de spermaceti.

Par ailleurs, la composition thérapeutique selon l'invention, quand elle se présente sous forme d'une émulsion telle que définie plus haut, peut également avantageusement comporter un ou plusieurs agents tensioactifs. Ce dernier présente un caractère lipophile ou hydrophile caractérisé par une valeur HLB (hydrophile-lipophile-balance) comprise entre 1 et 19.

Un tel tensioactif peut consister en :
* un alkylpolyglycoside ou un mélange d'alkylpolyglycosides de formule Ra-(0)- Zn où Ra représente un radical aliphatique saturé, linéraire ou ramifié, comprenant de 4 à 24 atomes de carbone, de préférence de 8 à 22 atomes de carbone, Z est le reste d'un sucre, de préférence le glucose et n est compris entre 1 et 5, de préférence entre 1,1 et 2,
* les saponines,
* les lécithines,
* les alcanols polyoxyéthylés, tels ceux commercialisés sous la désignation BRIJ par la société ICI,
* les polymères comprenant des blocs polyoxyéthylènes et polyoxypropylènes, tels ceux commercialisés sous la désignation PLURONICS par la société BASF.

Des tensioactifs particulièrement préférés sont les esters de polyethylèneglycol, obtenus par condensation d'un acide gras, notamment un acide gras liquide à 20°C, avec un polyethylèneglycol de poids moléculaire compris entre 80 et 2000 ; un tel tensioactif est commercialisé par la Société SEPPIC sous la marque SIMULSOL 2599.

Un autre agent tensioactif préféré dans le cadre de la présente invention consiste en un ester obtenu par condensation d'un acide gras, avantageusement un acide gras liquide à 20°C, avec un sucre, le sorbitol ou du glycérol. Ledit sucre peut consister en le glucose, le saccharose ou, de préférence, le mannitol. A titre d'ester de mannitol particulièrement préféré, on peut citer des oléates de mannitol obtenus par anhydrisation de la chaîne carbonée polyhydroxylée du mannitol qui se cyclise en 1-4 ou en 2-6.

Des dérivés de ces esters de sucre de polyéthylèneglycole, de sorbitol ou de glycérol peuvent être également mis en oeuvre. Ces dérivés présentent une hydrophilie modifiée notamment par greffage de fonctions hydrophiles telles alcool, polyol, oxyde d'éthylène, oxyde de propylène, acide carboxylique, amine, ou amide. De tels dérivés peuvent par exemple consister en les esters gras de sorbitanne polyoxyéthylés, tels les TWEEN (conf. l'International cosmetic Ingrédient Dictionary, 5^{éme} éd., 1993). Un autre type de tensioactif préféré consiste en les huiles végétales éthoxylés, telles que, par exemple, l'huile de ricin éthoxylée, cette huile étant hydrogénée ou non.

Un agent tensioactif selon l'invention est de préférence pharmaceutiquement acceptable pour un usage en injectable ; il doit être notamment dépourvu de métaux lourds et présenter des indices d'acides ou de peroxydes très faibles. Il est également souhaitable qu'il satisfasse les normes de tests d'innocuités tel que par exemple, ceux décrits par S.S. Berlin, Annales of Allergy, 1962, 20, 473 ou les tests de toxicité anormale décrits dans la pharmacopée européenne. Préférentiellement, l'agent tensioactif est associé à l'adjuvant huileux avant formation de l'émulsion.

La concentration en l'agent tensioactif dans la composition thérapeutique peut être comprise entre 0,01 et 500 mg/ml, de préférence entre 0,1 et 200 mg/ml.

Des huiles associées avec un agent tensioactif (ester de mannitol) convenant tout particulièrement dans le cadre de la présente invention sont celles commercialisées par la Société SEPPIC sous la marque "MONTANIDE". La nature de ces huiles, le type d'émulsion qu'elles permettent d'obtenir et les caractéristiques(viscosité et conductivité) de ces émulsions figurent dans le tableau 1 ci-après :

**Tableau 1 :**

| N° | Nom commercial | Huile/esters de mannitol | Type de l'émulsion | Phase aqueuse/émulsion (% en poids) | Viscosité (mPa.s) | Conductivité à 25°C (µS.cm⁻¹) |
|---|---|---|---|---|---|---|
| 1 | MONTANIDE ISA 25 | Minérale | H/E | 75 % | 20 | 5000 |
| 2 | MONTANIDE ISA 25A | Minérale + avridine | H/E | 75 % | 20 | 5000 |
| 3 | MONTANIDE ISA 28 | Minérale + oléate d'éthyle | H/E | 75 % | 25 | 1000 |
| 4 | MONTANIDE ISA 206 | Minérale | E/H/E | 50 % | 50 | 1000 |
| 5 | MONTANIDE ISA 50 | Minérale | E/H | 50 % | 200 | 1 |
| 6 | MONTANIDE ISA 708 | Végétale | E/H | 30 % | 70 | 1 |
| * Avridine = N,N-dioctadecyl-N', N'-bis-(2-hydroxyéthyl)-propanediamine. | | | | | | |

L'adjuvant huileux peut encore consister en une huile auto-émulsionnable, c'est-à-dire une préparation huileuse capable de former une émulsion stable avec une phase aqueuse, pratiquement sans apport d'énergie, par exemple par dispersion dans la phase aqueuse par agitation mécanique lente. A ce titre, on peut citer des huiles auto-émulsionnables telles que celles connues dans la pharmacopée européenne sous les désignations Labrafil et Simulsol. Ces huiles sont des glycérides polyglycosés.

Des huiles auto-émulsionnables préférées sont celles décrites dans la demande de brevet français N° 9500497 déposée le 18 Janvier 1995, au nom de la Demanderesse, intitulée "utilisation d'esters d'acides gras éthoxylés comme composants auto-émulsionnables notamment utiles pour la préparation de compositions phytosanitaires ou de médicaments à usage vétérinaire ou humain", dont référence est intégrée à la présente description. Ces huiles consistent en des esters d'acides gras éthoxylés répondant à l'une des formules suivantes : 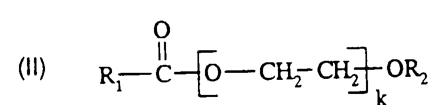 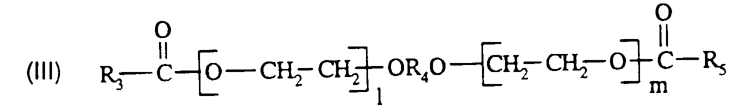 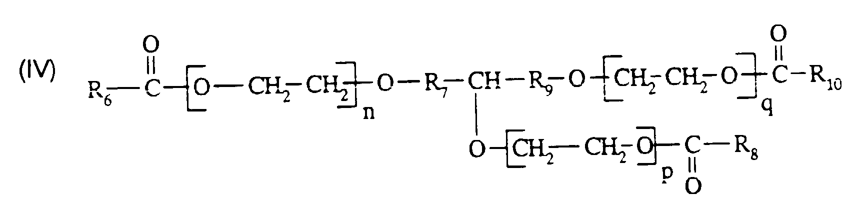 dans lesquelles :
- R₁, R₃, R₅, R₆, R₈ et R₁₀ représentent une chaîne hydrocarbonée, linéaire ou ramifiée, saturée ou insaturée ayant de 5 à 30 atomes de carbone ;
- R₂, R₄, R₇ et R₉ représentent une chaîne hydrocarbonée, linéaire ou ramifiée, saturée ou insaturée ayant de 1 à 5 atomes de carbone;
le nombre total de molécules d'oxyde d'éthylène respectivement représenté dans les formules II, III et IV précitées par k, l+m, n+p+q, étant un nombre entier tel que la valeur HLB (balance hydrophile-lipophile) desdits composés soit comprise entre environ 4 et environ 10, de préférence entre environ 5 et environ 9.

R₁ est de préférence choisi parmi les restes des acides palmitique, stéarique, ricinoléique, oléique, linoléique et linolénique et R₂ représente un radical méthyle et k est un nombre entier compris entre 1 et 5, de préférence égal à 2, et par ailleurs, les esters d'acides gras éthoxylés de formule III préférés sont ceux où :
(i) - R₆, R₈ et R₁₀ représentent des chaînes hydrocarbonées ayant de 16 à 22 atomes de carbone correspondant notamment aux chaînes grasses de l'huile de colza, de maïs, de soja, d'arachide et de noyaux d'abricots.
   - R₇ et R₉ représentent un groupe méthylène CH₂ ;
   - n, p, q représentent des nombres entiers tels que leur somme soit comprise entre 3 et 30, et de préférence égale à 20 ; ou
(ii) - R₆, R₈ et R₁₀ représentent des chaînes hydrocarbonées correspondant aux chaînes grasses de l'huile de ricin ;
   - R₇ et R₉ représentent un radical méthylène CH₂ ;
   - n, p, q représentent des nombres entiers tels que leur somme soit comprise entre 5 et 7.

La concentration en huile auto-émulsionnable dans la composition thérapeutique selon l'invention peut être comprise entre environ 5 et 700 g/l, de préférence entre environ 10 et 500 g/l.

Outre la phase huile et la phase aqueuse, la composition selon l'invention peut comporter un agent stimulant immunitaire conventionnel tel l'Avridine®, soit la N,N-dioctadecyl-N',N' - bis (2-hydroxyethyl) propanediamine, les dérivés du MDP (muramyl dipeptide) notamment le threonyl-MDP, les dérivés de l'acide mycolique ou les dérivés du Lipide A.

La composition thérapeutique selon l'invention peut comprendre encore un ou plusieurs agents tensioactifs, en l'absence de tout adjuvant huileux.

La composition thérapeutique se présente alors sous la forme d'une solution micellaire. Celle-ci peut être préparée par simple mélange de l'agent tensioactif avec une dispersion dans l'eau de l'antigène ou du générateur d'antigène in vivo.

L'agent tensioactif peut être choisi parmi les agents tensioactifs décrits plus haut, en association avec un adjuvant huileux.

Ladite solution micellaire peut comporter de 0,5 à 500 mg/ml, de préférence de 1 à 250 mg/ml en agent tensioactif.

Une composition thérapeutique selon la présente invention peut comprendre un antigène tel qu'un virus, un micro-organisme, plus particulièrement une bactérie ou un parasite, ou un composé comprenant une chaîne peptidique. Un tel composé peut consister en une protéine ou une glycoprotéine, notamment une protéine ou une glycoprotéine issues d'un micro-organisme, un peptide synthétique ou une protéine ou un peptide issu du génie génétique. Lesdits virus et micro-organisme peuvent être totalement inactivés ou vivants atténués. A titre de virus pouvant constituer un antigène selon la présente invention, on peut citer le virus de la rage, les virus Aujeszky, Influenzae, le virus de la fièvre aphteuse ou les VIH. A titre de micro-organisme du type bactérien pouvant constituer un antigène selon la présente invention, on peut citer E. coli et ceux des genres Pasteurella, Furonculosis, Vibriosis, Staphylococcus.et Streptococcus. A titre de parasite, on peut citer ceux des genres Trypanosoma, Plasmodium et Leishmania.

Une composition thérapeutique selon l'invention comprend une concentration en antigène qui dépend de la nature de cet antigène et de la nature du sujet traité. Il est toutefois particulièrement remarquable qu'un adjuvant selon l'invention, associé ou non à un adjuvant huileux et/ou un agent tensioactif tels que définis plus haut, permet de diminuer d'une façon notable la dose habituelle d'antigène requise. La concentration adéquate d'antigène peut être déterminée de manière classique par l'homme du métier. Généralement, cette dose est de l'ordre de 0,1µg/ml à 1g/ml plus généralement comprise entre 1 à µg/ml et 100mg/ml.

Une composition thérapeutique selon l'invention peut aussi comprendre un générateur in vivo d'un composé comprenant une séquence d'acides aminés, c'est-à-dire un composé biologique capable d'exprimer un tel composé dans l'organisme hôte dans lequel on a introduit ledit générateur in vivo. Le composé comprenant la séquence d'acides aminés, peut être une protéine, un peptide ou une glycoprotéine.

Ces générateurs in vivo sont généralement obtenus par des procédés issus du génie génétique.

Plus particulièrement, ils peuvent consister en des microorganismes vivants, généralement un virus, jouant le rôle de vecteur recombinant, dans lequel est inséré une séquence nucléotidique, notamment un gène exogène. Ces composés sont connus en tant que tels et utilisés notamment comme vaccin sous unitaire recombinant.

A cet égard, on peut se référer à l'Article de M. ELOIT et al., Journal of virology (1990) 71, 2925-2431, à la demande internationale WO-A-91.00107 ou à la demande internationale WO-A-94/16681.

Avantageusement, le micro-organisme constitutif d'un vaccin sous-unitaire recombinant est un virus recombinant non enveloppé, par exemple choisi parmi les adénovirus, le virus de la vaccine, le canarypox virus, les herpès virus ou les baculovirus. Le gène exogène inséré dans le micro-organisme peut être, par exemple, issu d'un virus Aujeszky ou HIV.

Les générateurs in vivo selon l'invention peuvent aussi consister en un plasmide recombinant comprenant une séquence nucléotidique exogène, capable d'exprimer dans un organisme hôte un composé comprenant une séquence d'acides aminés. De tels plasmides recombinants et leur mode d'administration dans un organisme hôte ont été décrits en 1990, par Lin et al., circulation 82:2217,2221 ; COX et al., J. of Virol, Sept. 1993, 67, 9, 5664-5667 et dans la demande internationale WO/FR 95/00345 du 21 Mars 1995, au nom de la demanderesse, intitulée "Une composition comprenant un plasmide recombinant et ses utilisations comme vaccin et médicament".

Selon la nature de la séquence nucléotidique comprise dans le générateur in vivo, le composé comprenant la séquence d'acides aminés qui est exprimé au sein de l'organisme hôte, peut :
(i) être un antigène, et permettre le déclenchement d'une réaction immune,
(ii) avoir une action curative vis-à-vis d'une maladie, essentiellement une maladie d'ordre fonctionnel, qui s'est déclenchée chez l'organisme hôte. Dans ce cas, le générateur in vivo permet un traitement thérapeutique de l'hôte, du type thérapie génique.

A titre d'exemple, une telle action curative peut consister en une synthèse par le générateur in vivo de cytokine, comme les interleukines, notamment l'interleukine 2. Celles-ci permettent le déclenchement ou le renforcement d'une réaction immune visant à l'élimination sélective des cellules cancéreuses.

La concentration en ledit générateur in vivo dans la composition thérapeutique selon l'invention dépend, là encore, notamment de la nature dudit générateur et de l'hôte dans lequel il est administré. Cette concentration peut être aisément déterminée par l'homme du métier, sur la base d'expériences de routine.

A titre indicatif, on peut toutefois préciser que lorsque le générateur in vivo est un microorganisme recombinant, sa concentration dans la composition thérapeutique selon l'invention peut être comprise entre 10² et 10¹⁵ microorganismes / ml, de préférence entre 10⁵ et 10¹² microorganismes /ml.

Lorsque le générateur in vivo est un plasmide recombinant, sa concentration dans la composition thérapeutique selon l'invention peut être comprise entre 0,01 et 100 g/l.

Une composition thérapeutique selon l'invention peut être utilisée comme médicament préventif ou curatif. Selon la nature de l'antigène ou du générateur in vivo, une composition thérapeutique selon l'invention peut être administrée à des poissons, des crustacés tels les crevettes, des volailles notamment, des oies, des dindes, des pigeons et des poulets, aux canidés tels le chien, aux félidés tels le chat, aux porcs, aux primates, aux bovidés, aux ovidés et aux chevaux. La composition thérapeutique selon l'invention comprenant un sel pharmaceutiquement acceptable soluble dans l'eau tel que défini ci-dessus, peut être également administrée à l'homme. L'administration de la composition thérapeutique peut se faire de manière classique, notamment par injection sous-cutanée, intramusculaire ou intrapéritonéale par voie orale ou par voie mucosale.

Selon un autre aspect de l'invention, celle-ci consiste en l'utilisation d'un adjuvant consistant en un sel pharmaceutiquement acceptable soluble dans l'eau tel que défini ci-dessus pour la préparation d'un vaccin destiné à la prévention ou au traitement d'une maladie infectieuse, notamment une maladie infectieuse engendrée par un virus ou un micro-organisme tels ceux mentionnés plus haut.

Selon un autre aspect de l'invention, celle-ci consiste en l'utilisation d'un sel parmaceutiquement acceptable soluble dans l'eau pour la préparation d'une compositon thérapeutique destiné à soigner une maladie d'ordre fonctionnelle, telle le cancer ou la mucoviscidose .

Dans l'une ou l'autre de ces utilisations, ledit sel pharmaceutiquement acceptable peut être associé à l'un au moins d'un adjuvant huileux, d'une agent tensioactif ou d'un adjuvant huileux lui-même associé à un agent tensioactif ; ces adjuvants huileux et tensioactifs étant tels que définis plus haut.

Des compositions adjuvantes comprenant ledit sel pharmaceutiquement acceptable et l'adjuvant huileux et/ou les tensioactifs précités constituent encore un autre aspect de l'invention. Le cas échéant, ces compositions adjuvantes comprennent au moins une phase aqueuse.

Dans ce dernier cas, les compositions adjuvantes selon l'invention, comprenant au moins un adjuvant huileux, et, le cas échéant, un tensioactif, peuvent se présenter sous la forme d'une émulsion. Cette dernière peut être du type E/H, H/E, E/H/E ou microémulsion.

Ces émulsions peuvent comprendre en poids, de 0,5 % à 99,5 % de phase huile pour 99,5 % à 0,5 % de phase aqueuse, de préférence, de 5 à 95 % de phase huile pour 95 à 5 % de phase aqueuse et, plus préférentiellement, de 25 à 75 % de phase huile pour 75 à 25 % de phase aqueuse.

Le cas échéant, elles peuvent comprendre de 0,01 à 500 mg/ml, de préférence de 0,1 à 200 mg/ml d'au moins un tensioactif.

Lorsque la composition adjuvante selon l'invention ne comprend, outre le sel pharmaceutiquement acceptable et une phase aqueuse, qu'un ou plusieurs tensioactifs, elle se présente alors sous la forme d'une solution micellaire. La teneur en tensioactif de cette solution micellaire peut être comprise entre 0,01 et 900 mg/ml, de préférence entre 1 et 250 mg/ml.

Une composition adjuvante selon l'invention comprend habituellement de 0,02 à 3000 mg/ml, de préférence 0,1 à 1000 mg/ml, plus préférentiellement de 0,1 à 150 mg/ml d'un sel pharmaceutiquement acceptable selon l'invention.

Ces compositions adjuvantes sont utiles pour préparer les compositions thérapeutiques selon l'invention.

Ces dernières peuvent alors être préparées par simple mélange de la composition adjuvante avec une composition comprenant un antigène ou un générateur in vivo d'un composé comprenant une séquence d'acides aminés.

L'invention sera mieux comprise au regard des exemples et des figures ci-après.

Les exemples ont été réalisés sur des souris OF1 dont le poids moyen était compris entre 18 et 20 g.

Les résultats exprimés sont une moyenne des résultats obtenus sur 10 souris.

Les compositions thérapeutiques comprenaient, à titre d'antigène, de la sérum albumine bovine (BSA), Grade V, Sigma.

Les compositions thérapeutiques ont été injectées par voie sous-cutanée.

La réponse immunitaire humorale a été déterminée par dosage des IgG totales et des IgG₁, selon la méthode ELISA.

La réponse immunitaire cellulaire a été déterminée par dosage des IgG₂a, selon la méthode ELISA.

Les titres anticorps exprimés dans les exemples, correspondent à la dernière dilution au-dessus du bruit de fond.

Les abréviations suivantes sont utilisées dans les exemples :
- Glu = gluconate
- Fruhp = fructoheptonate
- Gly = glycérophosphate
- Gluhp = glucoheptonate
- Al(OH)₃ = hydroxyde d'aluminium
- Asp = acide aspartique
Ainsi, à titre d'exemple, GluMn est le gluconate de manganèse.

### Exemple 1

On prépare des compositions thérapeutiques (ou doses) de 100 µl chacune, comprenant 50 µg de BSA.

Ces compositions thérapeutiques ont été injectées chez les souris à JO (soit le jour de l'injection).

On a déterminé le taux d'anticorps anti-BSA à 14 et 28 jours après l'injection (réponse primaire).

A J28, on a injecté une même composition thérapeutique aux mêmes souris.

On a déterminé le taux d'anticorps anti-BSA à J42 et J56.

Les résultats obtenus figurent dans le tableau II ci-dessous.

**Tableau II :**

| Compositions vaccinales | Adjuvant | Doses d'adjuvant (en mg/dose) | Anticorps anti-BSA / réponse primaire (J14) | Anticorps anti-BSA / réponse primaire (J28) | Anticorps anti-BSA / réponse primaire (J42) | Anticorps anti-BSA / réponse secondaire (J56) |
|---|---|---|---|---|---|---|
| 1 | - | - | < 500 | < 500 | < 500 | < 500 |
| 2 | GluMn | 1 | 64000 | 32000 | 256000 | 256000 |
| 3 | GluCa | 3 | 16000 | 8000 | 128000 | 96000 |
| 4 | FruhpCa | 1 | 4000 | 2000 | 64000 | 48000 |
| 5 | GlyCa | 2 | 4000 | 2000 | 64000 | 64000 |
| 6 | GluZn | 0,5 | 16000 | 6000 | 96000 | 96000 |
| 7 | GluhpZn | 1 | 8000 | 6000 | 64000 | 64000 |
| 8 | AspMg | 1 | < 500 | < 500 | < 500 | < 500 |
| 9 | Asp Mg/K | 1 | < 500 | < 500 | < 500 | < 500 |
| 10 | Asp Ca | 1 | 4000 | 4000 | 48000 | 48000 |
| 11 | Asp Mn | 1 | 32000 | 32000 | 128000 | 128000 |
| | Témoin | - | < 500 | < 500 | < 500 | < 500 |

La composition témoin correspond aux souris qui n'ont pas été vaccinées.

Les résultats obtenus montrent que l'addition des adjuvants selon l'invention dans une composition thérapeutique induit une activité immuno-stimulante significative par rapport au témoin non vacciné et aux compositions thérapeutiques ne comprenant pas d'adjuvant.

Ces résultats montrent également la nette supériorité des sels divalents du zinc, du manganèse et du calcium vis-à-vis de l'aspartate de magnésium qui n'induit pas une activité immuno-stimulante significative par rapport au témoin non vacciné ou aux compositions ne comprenant pas d'adjuvant.

On peut aussi noter que le GluMn, bien qu'utilisé à une concentration faible, permet l'obtention d'un taux d'anticorps particulièrement élevé.

### Exemple 2

On a comparé l'effet immunostimulant d'un sel soluble dans l'eau selon l'invention, GluMn, avec celui d'un sel insoluble, Al(OH)₃.

Les compositions thérapeutiques (dose/souris) d'un volume de 100 µl mises en oeuvre comprenaient 50 µg d'antigène et 1 mg d'Al(OH)₃.

On a également injecté des doses ne comprenant pas d'adjuvant.

Un lot de souris n'a pas été vacciné (témoin).

Les résultats obtenus figurent dans le tableau III ci-dessous.

**Tableau III :**

| Adjuvant | IgG1 réponse primaire | IgG1 réponse primaire | IgG1 réponse secondaire | IgG1 réponse secondaire | IgG2a réponse primaire | IgG2a réponse primaire | IgG2a réponse secondaire | IgG2a réponse secondaire |
|---|---|---|---|---|---|---|---|---|
| | J14 | J28 | J42 | J56 | J14 | J28 | J42 | J56 |
| - | < 500 | < 500 | < 500 | < 500 | < 500 | < 500 | < 500 | < 500 |
| GluMn | 32000 | 64000 | 256000 | 256000 | 4000 | 8000 | 24000 | 64000 |
| Al(OH)₃ | 16000 | 64000 | 128000 | 256000 | 1000 | 6000 | 8000 | 32000 |
| Témoin | < 500 | < 500 | < 500 | < 500 | < 500 | < 500 | < 500 | < 500 |

Les IgG1 sont représentatifs de la réponse humorale.

Les IgG2 sont représentatifs de la réponse cellulaire.

Les réponses humorales et cellulaires obtenues avec un adjuvant selon l'invention sont supérieures à celles obtenues avec un adjuvant conventionnel tel Al(OH)₃.

### Exemple 3

Afin de mettre en évidence l'effet de synergie entre un adjuvant du type sel soluble dans l'eau avec un adjuvant huileux (le Montanide ISA 25 défini dans le tableau I), on a comparé l'effet de diverses compositions thérapeutiques. Chacune d'elles comprenait 50 µg d'antigène et avait un volume de 100 µl (dose/souris).

Les résultats obtenus figurent dans le tableau IV ci-dessous :

**Tableau IV :**

| Sel soluble dans l'eau | Quantité de sel (mg/dose) | Montanide ISA25 (en mg/dose) | Immunité humorale (J14) | Immunité cellulaire (J14) |
|---|---|---|---|---|
| - | - | 25 | 24000 | 3000 |
| GluMn | 1 | - | 64000 | 4000 |
| GluMn | 1 | 25 | 96000 | 8000 |
| GluMn | 0,5 | - | 16000 | < 2000 |
| GluMn | 0,5 | 25 | 96000 | 6000 |

L'association d'un adjuvant huileux à un sel soluble selon l'invention permet l'obtention d'un titre anticorps supérieur à celui obtenu par la simple addition des titres anticorps obtenu avec chacun de ces adjuvants mis en oeuvre individuellement. Une synergie est bien démontrée. Cette synergie est encore plus fortement marquée pour une quantité faible (0,5 mg/dose) de GluMn.

### Exemple 4

Pour démontrer l'importance du caractère organique de l'anion, on a comparé l'effet immunostimulant de différents sels de calcium.

Chaque composition thérapeutique, d'un volume de 100 µl contenait 50 µg d'antigène et, à l'exception du témoin,0,5 mg d'un sel de calcium, de sorte que la concentration en calcium de chaque composition soit de 2,7 mg.

Le dosage des anticorps a été effectué 42 jours après la vaccination.

L'injection de la composition thérapeutique a été répétée à J28. Le dosage des anticorps a été effectué à J42.

Les résultats obtenus figurent dans le tableau V :

**Tableau V :**

| Sels | Réponse humorale | Réponse cellulaire |
|---|---|---|
| CaCO₃ | 32000 | 4000 |
| CaCl₂ | 64000 | 24000 |
| CaHPO₄ | 64000 | 10000 |
| GluCa | 128000 | 32000 |
| - | 3000 | < 2000 |

Il a été constaté au cours de ces tests que le CaCl₂ induit des lésions très importantes.

### Exemple 5

On a injecté à différents lots comprenant chacun 5 souris, une composition thérapeutique d'un volume de 100 µl, comprenant 50 µg d'antigène. On a évalué les réactions locales (lésions et granulomes) à J8 et J35.

Les résultats obtenus figurent dans les tableaux VI et VII, respectivement.

**Tableau VI (J8) :**

| | Souris N° | 1 | 2 | 3 | 4 | 5 |
|---|---|---|---|---|---|---|
| | Concentrantion en sel (mg/dose | | | | | |
| GluMn | 1 | + | + | - | - | + |
| GlyMn | 1 | L | + | ++ | + | - |
| GluMn(*) | 1 | ++ | +++ | + | - | + |
| GluCa | 1 | - | - | - | - | - |
| MnCl2+ | 1 | +++ | L | L | L | L |
| Al(OH)3 | 1 | +++ | +++ | ++ | +++ | ++++ |

**Tableau VII (J35) :**

| | Souris N° | 1 | 2 | 3 | 4 | 5 |
|---|---|---|---|---|---|---|
| | Concentrantion en sel (mg/dose | | | | | |
| GluMn | 1 | - | + | + | + | - |
| GlyMn | 1 | - | + | + | + | + |
| GluMn(*) | 1 | ++ | ++ | ++ | +++ | + |
| GluCa | 1 | - | - | - | - | - |
| MnCl2+ | 1 | +++ | LL | LL | L | L |
| Al(OH)3 | 1 | ++++ | +++ | ++ | +++ | ++++ |

| | | | | | | |
|---|---|---|---|---|---|---|
| (*) : GluMn associé à 25 mg de MONTANIDE ISA 25 - = pas de granulome + = petit granulome ++ = granulome moyen +++ = granulome important ++++ = granulome très important - = pas de lésions L = petite lésion LL = lésion moyenne LLL = lésion importante LLLL = lésion très importante DCD = souris mortes pendant l'expérience. | | | | | | |

La concentration en Al(OH)₃ choisie correspond à la concentration à laquelle ce sel a permis l'obtention d'une réponse immunitaire la plus importante. Cette concentration en Al(OH)₃ est celle mise en oeuvre dans les exemples ci-dessus.

## Revendications

1. Composition thérapeutique comprenant (i) au moins un antigène ou au moins un générateur in vivo d'un composé comprenant une séquence d'acides aminés et (ii) au moins un adjuvant, caractérisée en ce que ledit adjuvant consiste en au moins un sel pharmaceutiquement acceptable et soluble dans l'eau, ledit sel étant constitué d'un anion organique et d'un cation métallique divalent choisi dans le groupe constitué par le manganèse, le calcium et le zinc.

2. Composition thérapeutique selon la revendication 1, caractérisée en ce que le cation métallique divalent est le manganèse .

3. Composition thérapeutique selon l'une des revendications 1 ou 2, caractérisée en ce que l'anion organique est dérivé d'un composé comprenant au moins un groupe fonctionnel oxygéné

4. Composition thérapeutique selon la revendication 3, caractérisée en ce que le groupe fonctionnel est un groupe carboxylique ou un groupe phosphorique.

5. Composition thérapeutique selon la revendication 4, caractérisée en ce que l'anion organique est dérivé d'un composé choisi dans le groupe constitué par les acides aminés, les oses acides comprenant de 5 à 7 atomes de carbone, et l'acide glycérophosphorique.

6. Composition thérapeutique selon la revendication 5, caractérisée en ce que l'acide aminé est l'acide aspartique.

7. Composition thérapeutique selon la revendication 6, caractérisée en ce que l'ose acide est choisi dans le groupe constitué par l'acide gluconique, l'acide fructoheptonique et l'acide glucoheptonique.

8. Composition thérapeutique selon l'une des revendications 1 à 5, caractérisée en ce que le sel pharmaceutiquement acceptable est le gluconate de manganèse.

9. Composition thérapeutique selon l'une des revendications 1 à 8, caractérisée en ce qu'elle se présente sous la forme d'une émulsion comprenant au moins une phase aqueuse et au moins une phase huile, la phase huile étant constituée par un adjuvant.

10. Composition thérapeutique selon la revendication 9, caractérisée en ce que l'émulsion est du type E/H/E, H/E ou microémulsion.

11. Composition thérapeutique selon l'une des revendications 9 et 10, caractérisée en ce que l'adjuvant huileux est associé à un agent tensioactif, de préférence un agent tensioactif consistant en (i) un ester obtenu par condensation d'un acide gras avec un sucre, un polyéthylèneglycol, le sorbitol du glycérol, ou un dérivé d'un tel ester dont l'hydrophilie a été modifiée et (ii) une huile végétale éthoxylée.

12. Composition thérapeutique selon l'une des revendications 9 et 10, caractérisée en ce que l'adjuvant huileux est une huile auto-émulsionnable.

13. Composition thérapeutique selon la revendication 12, caractérisée en ce que l'adjuvant huileux est un ester d'acides gras éthoxylés répondant à l'une des formules suivantes : 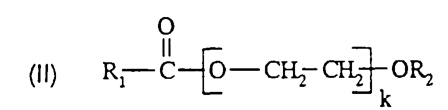 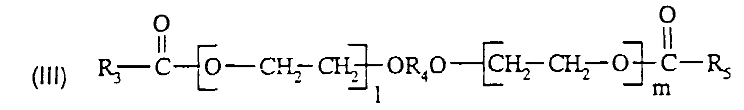 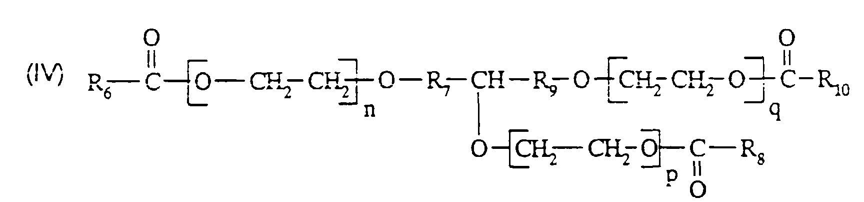 dans lesquelles :
- R₁, R₃, R₅, R₆, R₈ et R₁₀ représentent une chaîne hydrocarbonée, linéaire ou ramifiée, saturée ou insaturée ayant de 5 à 30 atomes de carbone ;
- R₂, R₄, R₇ et R₉ représentent une chaîne hydrocarbonée, linéaire ou ramifiée, saturée ou insaturée ayant de 1 à 5 atomes de carbone;
le nombre total de molécules d'oxyde d'éthylène respectivement représenté dans les formules II, III et IV précitées par k, l+m, n+p+q, étant un nombre entier tel que la valeur HLB (balance hydrophile-lipophile) desdits composés soit comprise entre environ 4 et environ 10, de préférence entre environ 5 et environ 9.

14. Composition thérapeutique selon l'une des revendications 1 à 8, caractérisée en ce qu'elle comprend en outre un agent tensioactif, ladite composition se présentant sous la forme d'une solution micellaire.

15. Composition thérapeutique selon la revendication 14, caractérisée en ce que l'agent tensioactif est (i) un ester obtenu par condensation d'un acide gras avec un sucre ou du glycérol, ou un dérivé d'un tel ester dont l'hydrophilie a été modifiée ou (ii) une huile végétale éthoxylée

16. Utilisation d'un sel pharmaceutiquement acceptable et soluble dans l'eau tel que défini dans l'une des revendications 1 à 8, pour la préparation d'une composition thérapeutique destinée à la prévention ou au traitement des maladies infectieuses.

17. Utilisation selon la revendication 16, caractérisée en ce que le sel est associé à un composé choisi dans le groupe constitué par un adjuvant huileux, un tensioactif et un adjuvant huileux associé à un agent tensio-actif.

18. Utilisation d'un sel pharmaceutiquement acceptable et soluble dans l'eau tel que défini dans l'une des revendications 1 à 8, pour la préparation d'une compositon thérapeutique destinée à soigner une maladie fonctionnelle.

19. Utilisation selon la revendication 18, caractérisée en ce que ledit sel est associé à un composé choisi dans le groupe constitué par un adjuvant huileux, un agent tensioactif et un adjuvant huileux associé à un agent tensioactif.

20. Composition adjuvante comprenant un sel pharmaceutiquement acceptable et soluble dans l'eau tel que défini dans l'une des revendications 1 à 8, et au moins un composé choisi dans le groupe constitué par, un adjuvant huileux, untensioactif et un adjuvant huileux associé à un tensioactif.

21. Composition selon la revendication 20, caractérisée en ce qu'elle comprend en outre au moins une phase aqueuse.

22. Composition selon la revendication 21, caractérisée en ce qu'elle se présente sous la forme d'une émulsion.

23. Composition selon la revendication 22, caractérisée en ce qu'elle se présente sous la forme d'une solution micellaire.

## Patentansprüche

1. Therapeutische Zusammensetzung enthaltend (i) mindestens ein Antigen oder mindestens einen in vivo-Erzeuger einer Verbindung mit einer Aminosäuresequenz sowie (ii) mindestens einen Hilfsstoff, dadurch gekennzeichnet, daß dieser Hilfsstoff aus mindestens einem pharmazeutisch unbedenklichen, wasserlöslichen Salz besteht, wobei dieses Salz aus einem organischen Anion und einem zweiwertigen Metallkation aus der Gruppe Mangan, Calcium und Zink besteht.

2. Therapeutische Zusammensetzung nach Anspruch 1, dadurch gekennzeichnet, daß es sich bei dem zweiwertigen Metallkation um Mangan handelt.

3. Therapeutische Zusammensetzung nach Anspruch 1 oder 2, dadurch gekennzeichnet, daß sich das organische Anion von einer Verbindung mit mindestens einer sauerstoffhaltigen funktionellen Gruppe ableitet.

4. Therapeutische Zusammensetzung nach Anspruch 3, dadurch gekennzeichnet, daß es sich bei der funktionellen Gruppe um eine Carboxylgruppe oder eine Phosphorgruppe handelt.

5. Therapeutische Zusammensetzung nach Anspruch 4, dadurch gekennzeichnet, daß sich das organische Anion von einer Verbindung aus der Gruppe Aminosäuren, saure Osen mit 5 bis 7 Kohlenstoffatomen sowie Glycerophosphorsäure ableitet.

6. Therapeutische Zusammensetzung nach Anspruch 5, dadurch gekennzeichnet, daß es sich bei der Aminosäure um Asparaginsäure handelt.

7. Therapeutische Zusammensetzung nach Anspruch 6, dadurch gekennzeichnet,- daß die saure Ose aus der Gruppe Gluconsäure, Fructoheptonsäure und Glucoheptonsäure stammt.

8. Therapeutische Zusammensetzung nach einem der Ansprüche 1 bis 5, dadurch gekennzeichnet, daß es sich bei dem pharmazeutisch unbedenklichen Salz um Mangangluconat handelt.

9. Therapeutische Zusammensetzung nach einem der Ansprüche 1 bis 8, dadurch gekennzeichnet, daß sie in Form einer Emulsion mit mindestens einer wäßrigen Phase und mindestens einer Ölphase vorliegt, wobei die Ölphase aus einem Hilfsstoff besteht.

10. Therapeutische Zusammensetzung nach Anspruch 9, dadurch gekennzeichnet, daß die Emulsion den Typ W/O/W, O/W oder den Typ einer Mikroemulsion aufweist.

11. Therapeutische Zusammensetzung nach Anspruch 9 oder 10, dadurch gekennzeichnet, daß der ölige Hilfsstoff mit einem Tensid, vorzugsweise einem Tensid bestehend aus (i) einem durch Kondensation einer Fettsäure mit einem Zucker, einem Polyethylenglykol, Sorbit oder Glycerin erhaltenen Ester oder einem Derivat eines solchen Esters, dessen Hydrophilität modifiziert wurde, sowie (ii) einem ethoxylierten pflanzlichen Öl zusammengegeben wird.

12. Therapeutische Zusammensetzung nach Anspruch 9 oder 10, dadurch gekennzeichnet, daß es sich bei dem öligen Hilfsstoff um ein selbstemulgierbares Öl handelt.

13. Therapeutische Zusammensetzung nach Anspruch 12, dadurch gekennzeichnet, daß es sich bei dem öligen Hilfsstoff um einen Ester von ethoxylierten Fettsäuren handelt, der einer der folgenden Formeln entspricht: 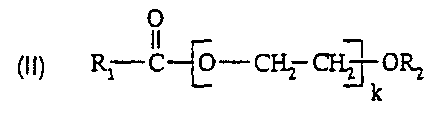 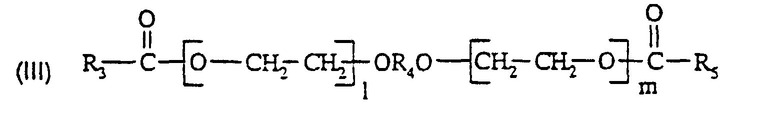 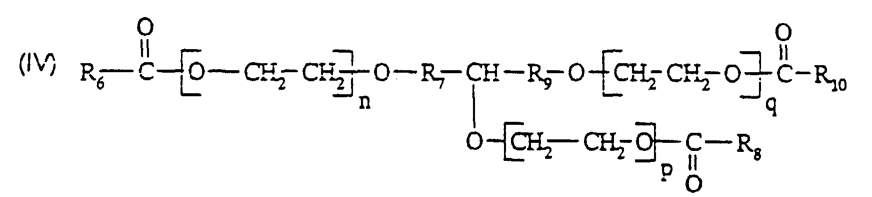 in denen:
- R₁, R₃, R₅, R₆, R₈ und R₁₀ eine gesättigte oder ungesättigte geradkettige oder verzweigte Kohlenwasserstoffkette mit 5 bis 30 Kohlenstoffatomen darstellen,
- R₂, R₄, R₇ und R₉ eine gesättigte oder ungesättigte, geradkettige oder verzweigte Kohlenwasserstoffkette mit 1 bis 5 Kohlenstoffatomen darstellen;
wobei die Gesamtzahl der Ethylenoxidmoleküle, die in den genannten Formeln II, III und IV durch k, l+m bzw. n+p+q bezeichnet werden, eine ganze Zahl ist, und zwar derart, daß der HLB-Wert (hydrophilic-lipophilic balance) der Verbindungen zwischen ungefähr 4 und ungefähr 10, vorzugsweise zwischen ungefähr 5 und ungefähr 9, liegt.

14. Therapeutische Zusammensetzung nach einem der Ansprüche 1 bis 8, dadurch gekennzeichnet, daß sie weiterhin ein Tensid enthält, wobei die Zusammensetzung als Mizellenlösung vorliegt.

15. Therapeutische Zusammensetzung nach Anspruch 14, dadurch gekennzeichnet, daß es sich bei dem Tensid um (i) einen durch Kondensation einer Fettsäure mit einem Zucker oder Glycerin erhaltenen Ester oder um ein Derivat solch eines Esters, dessen Hydrophilität modifiziert wurde, oder (ii) um ein ethoxyliertes pflanzliches Öl handelt.

16. Verwendung eines pharmazeutisch unbedenklichen, wasserlöslichen Salzes nach einem der Ansprüche 1 bis 8 zur Herstellung einer therapeutischen Zusammensetzung zur Vorbeugung - oder Behandlung von Infektionskrankheiten.

17. Verwendung nach Anspruch 16, dadurch gekennzeichnet, daß das Salz mit einer Verbindung aus der Gruppe ölige Hilfsstoffe, Tenside und deren Kombinationen zusammengegeben wird.

18. Verwendung eines pharmazeutisch unbedenklichen, wasserlöslichen Salzes nach einem der Ansprüche 1 bis 8 zur Herstellung einer therapeutischen Zusammensetzung zur Behandlung einer Funktionskrankheit.

19. Verwendung nach Anspruch 18, dadurch gekennzeichnet, daß dieses Salz mit einer Verbindung aus der Gruppe ölige Hilfsstoffe, Tenside und deren Kombinationen zusammengegeben wird.

20. Hilfsstoffzusammensetzung enthaltend ein pharmazeutisch unbedenkliches, wasserlösliches Salz nach einem der Ansprüche 1 bis 8 und mindestens eine Verbindung aus der Gruppe ölige Hilfsstoffe, Tenside und deren Kombinationen.

21. Zusammensetzung nach Anspruch 20, dadurch gekennzeichnet, daß sie außerdem mindestens eine wäßrige Phase enthält.

22. Zusammensetzung nach Anspruch 21, dadurch gekennzeichnet, daß sie in Form einer Emulsion vorliegt.

23. Zusammensetzung nach Anspruch 22, dadurch gekennzeichnet, daß sie in Form einer Mizellenlösung vorliegt.

## Claims

1. Therapeutic composition comprising (i) at least one antigen or at least one in vivo generator of a compound comprising an amino acid sequence and (ii) at least one adjuvant, characterized in that the said adjuvant consists of at least one pharmaceutically acceptable and water-soluble salt, the said salt consisting of an organic anion and a divalent metal cation chosen from the group consisting of manganese, calcium and zinc.

2. Therapeutic composition according to Claim 1, characterized in that the divalent metal cation is manganese.

3. Therapeutic composition according to either of Claims 1 and 2, characterized in that the organic anion is derived from a compound comprising at least one oxygenated functional group.

4. Therapeutic composition according to Claim 3, characterized in that the functional group is a carboxylic group or a phosphoric group.

5. Therapeutic composition according to Claim 4, characterized in that the organic anion is derived from a compound chosen from the group consisting of amino acids, acid saccharides comprising from 5 to 7 carbon atoms, and glycerophosphoric acid.

6. Therapeutic composition according to Claim 5, characterized in that the amino acid is aspartic acid.

7. Therapeutic composition according to Claim 6, characterized in that the acid saccharide is chosen from the group consisting of gluconic acid, fructoheptonic acid and glucoheptonic acid.

8. Therapeutic composition according to one of Claims 1 to 5, characterized in that the pharmaceutically acceptable salt is manganese gluconate.

9. Therapeutic composition according to one of Claims 1 to 8, characterized in that it is in the form of an emulsion comprising at least one aqueous phase and at least one oil phase, the oil phase consisting of an adjuvant.

10. Therapeutic composition according to Claim 9, characterized in that the emulsion is of the W/O/W, O/W or microemulsion type.

11. Therapeutic composition according to either of Claims 9 and 10, characterized in that the oily adjuvant is combined with a surface-active agent, preferably a surface-active agent consisting of (i) an ester obtained by condensation of a fatty acid with a sugar, a polyethylene glycol, sorbitol, glycerol, or a derivative of such an ester whose hydrophilicity has been modified, and (ii) an ethoxylated plant oil.

12. Therapeutic composition according to either of Claims 9 and 10, characterized in that the oily adjuvant is a self-emulsifiable oil.

13. Therapeutic composition according to Claim 12, characterized in that the oily adjuvant is an ester of ethoxylated fatty acids corresponding to one of the following formulae: 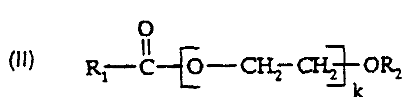 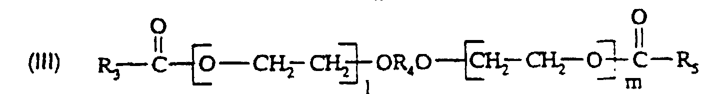 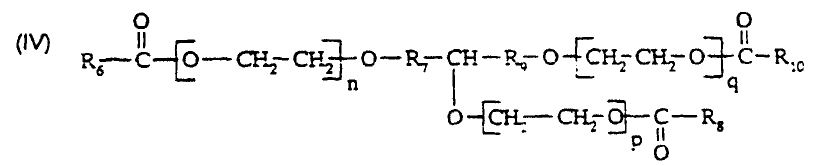 in which
- R₁, R₃, R₅, R₆, R₈ and R₁₀ represent a saturated or unsaturated, linear or branched hydrocarbon chain having from 5 to 30 carbon atoms;
- R₂, R₄, R₇ and R₉ represent a saturated or unsaturated, linear or branched hydrocarbon chain having from 1 to 5 carbon atoms;
the total number of ethylene oxide molecules represented in the abovementioned formulae II, III and IV by k, l+m and n+p+q, respectively, being an integer such that the HLB (hydrophilic-lipophilic balance) value of the said compounds is between about 4 and about 10, preferably between about 5 and about 9.

14. Therapeutic composition according to one of Claims 1 to 8, characterized in that it additionally comprises a surface-active agent, the said composition being in the form of a micellar solution.

15. Therapeutic composition according to Claim 14, characterized in that the surface-active agent is (i) an ester obtained by condensation of a fatty acid with a sugar or glycerol, or a derivative of such an ester whose hydrophilicity has been modified, or (ii) an ethoxylated plant oil.

16. Use of a pharmaceutically acceptable and water-soluble salt as defined in one of Claims 1 to 8 for the preparation of a therapeutic composition intended for the prevention or treatment of infectious diseases.

17. Use according to Claim 16, characterized in that the salt is combined with a compound chosen from the group consisting of an oily adjuvant, a surfactant and an oily adjuvant combined with a surface-active agent.

18. Use of a pharmaceutically acceptable and water-soluble salt as defined in one of Claims 1 to 8 for the preparation of a therapeutic composition intended to treat a functional disease.

19. Use according to Claim 18, characterized in that the said salt is combined with a compound chosen from the group consisting of an oily adjuvant, a surface-active agent and an oily adjuvant combined with a surface-active agent.

20. Adjuvant composition comprising a pharmaceutically acceptable and water-soluble salt as defined in one of Claims 1 to 8, and at least one compound chosen from the group consisting of an oily adjuvant, a surfac-tant and an oily adjuvant combined with a surfactant.

21. Composition according to Claim 20, characterized in that it additionally comprises at least one aqueous phase.

22. Composition according to Claim 21, characterized in that it is in the form of an emulsion.

23. Composition according to Claim 22, characterized in that it is in the form of a micellar solution.
